# FASCICULE DE BREVET EUROPEEN

(11) **EP 3 171 795 B1**
(45) Date de publication et mention de la délivrance du brevet: **07.11.2018**
(21) Numéro de dépôt: 15753125.2
(22) Date de dépôt: 24.07.2015
(51) Int. Cl.: A61B 17/15

(54) **PROCÉDÉ DE CONCEPTION D'UN DISPOSITIF CHIRURGICAL D'AIDE A LA POSE D'UN IMPLANT ORTHOPEDIQUE ENTRE DEUX OS D'UNE ARTICULATION D'UN PATIENT**
ENTWURFSPROZESS EINES CHIRURGISCHES INSTRUMENTS ZUR FÜHRUNG DER PLAZIERUNG EINES ORTHOPÄDISCHEN IMPLANTATS ZWISCHEN ZWEI KNOCHEN EINES GELENKS EINES PATIENTEN
DESIGN PROCESS OF A SURGICAL DEVICE FOR GUIDING THE POSITIONING OF AN ORTHOPAEDIC IMPLANT BETWEEN TWO BONES OF A PATIENT'S JOINT

(30) Priorité: 24.07.2014 FR 1457166
(43) Date de publication de la demande: 31.05.2017
(73) Titulaire: One Ortho, 69230 Saint-Genis-Laval (FR)
(72) Inventeur: ALEPEE, Christophe, 69003 Lyon (FR); GUITON, Thierry, 76100 Rouen (FR)
(74) Mandataire: Cabinet Laurent & Charras
(86) Numéro de dépôt international: PCT/FR2015/052053
(87) Numéro de publication internationale: WO 2016/012731

(56) Documents cités:
- US-A1- 2005 143 746
- US-A1- 2005 149 037
- US-A1- 2009 088 760
- US-B1- 6 478 799

## Description

La présente invention se rapporte au secteur technique de la chirurgie orthopédique, et concerne plus particulièrement un procédé de conception d'un dispositif chirurgical d'aide à la pose d'un implant orthopédique entre deux os d'une articulation d'un patient.

L'invention trouve une application pour tout type d'implant d'articulation orthopédique telles que prothèses du genou, prothèses d'épaule et, plus généralement, tout type d'implant orthopédique nécessitant de réaliser des coupes au niveau de la zone osseuse anatomique devant être prothésée.

Il est parfaitement connu pour un homme du métier d'utiliser différents dispositifs, communément appelés ancillaires de pose pour la mise en place de différents implants orthopédiques. Par exemple, les dispositifs peuvent être constitués par des blocs de guidage tibial, des blocs de guidage fémoral ...Ces blocs intègrent des fentes dans leur épaisseur pour le passage et le guidage d'instruments à lame de coupe, lesquelles fentes sont convenablement orientées et positionnées en fonction des coupes osseuses qu'il convient de réaliser pour la pose de l'implant.

Par exemple, dans le cas de l'arthrose du genou, le cartilage de l'articulation s'use jusqu'à générer une déformation angulaire du membre comprenant l'articulation. Par exemple, une déformation du genou en valgus se caractérise par une usure articulaire au niveau du compartiment externe du genou. Le centre du genou se retrouve très éloigné de l'axe mécanique de la jambe. Ainsi, pour pallier à ce problème, il faut insérer une « cale » au niveau de la partie usée pour combler l'espace défini par l'usure et ré-axer automatiquement le genou.

Pour effectuer une telle opération, il est connu de l'état de la technique de réaliser en premier lieu une coupe osseuse tibiale, puis de positionner dans l'espace généré par la coupe un organe de stabilisation intra-articulaire, dit « spacer », jouant le rôle de la « cale » susmentionnée, afin de réaligner le membre inférieur.

Après cette opération, on réalise en second lieu une coupe distale du fémur, puis on contrôle l'espace entre les deux os ainsi obtenu. Ainsi, selon l'état de la technique actuel, on réalise une première coupe, puis on oriente et on règle la position de la seconde coupe par rapport à la première. De ce fait, le contrôle se fait à postériori, avec des coupes indépendantes les unes des autres, ce qui laisse place à des imprécisions induites par ce mode opératoire. De plus, le balancement ligamentaire résultant des coupes est imprécis, et dois souvent être rectifié. Cela entraine parfois une coupe sur une partie du tibia plus importante que nécessaire, au risque de décaler l'interligne articulaire.

US2009/088760 A1 divulgue un procédé de conception d'un dispositif chirurgical comprenant les caractéristiques du préambule de la revendication 1.

L'un des buts de l'invention est donc de proposer un procédé de conception d' un dispositif chirurgical d'aide à la pose d'un implant orthopédique entre deux os d'une articulation d'un patient, qui permette de remédier au moins à l'un des inconvénients précités.

Le dispositif chirurgical comprend un organe de stabilisation intra-articulaire, destiné à être positionné entre les deux os de l'articulation pour stabiliser et ré-axer un membre comprenant l'articulation en comblant un espace défini par une usure d'une portion de l'articulation, et deux blocs de guidage fixés à l'organe de stabilisation, dont au moins l'un d'une manière amovible, et de part et d'autre d'un plan défini par ledit organe de stabilisation. Les blocs de guidage comprennent des moyens de fixation à l'os et présentent chacun au moins une fente pour l'engagement et le guidage d'un instrument destiné à réaliser des opérations de coupes de parties des os.

Ainsi, est divulgué un dispositif d'aide à la pose d'un implant orthopédique entre deux os d'une articulation d'un patient, dans lequel la ou lesdites fentes de l'un des blocs de guidage comprennent des orientations et des positions déterminées par rapport à la ou aux fentes de l'autre bloc de guidage. Le dispositif permet, en outre, d'aligner correctement les deux os de l'articulation et de positionner simultanément toutes les coupes osseuses à réaliser. De plus, les plans de coupes définis par les fentes d'un bloc de guidage sont orientés et positionnés relativement par rapport aux plans de coupes de l'autre bloc de guidage. La position relative des coupes les unes par rapport aux autres est parfaite et correctement balancée entre les deux os, ce qui assure une restitution optimale de l'interligne articulaire. Un seul dispositif pour mettre en place en une seule opération tous les plans de coupes nécessaires est mis en oeuvre. L' organe de stabilisation intra-articulaire est positionné sans qu'il soit nécessaire de réaliser une coupe osseuse préalable. C'est l'organe de stabilisation intra-articulaire qui est positionné pour combler un espace défini par l'usure d'une portion de l'articulation et permet ainsi de stabiliser et de ré-axer le membre comprenant l'articulation. Le positionnement de l'organe de stabilisation permet de positionner directement les coupes osseuses de manière adéquate.

De plus, le fait que l'un au moins des blocs de guidage, et de préférence les deux, soit fixé d'une manière amovible à l'organe de stabilisation intra-articulaire permet, après avoir positionné l'organe de stabilisation entre les deux os de l'articulation, et fixé les blocs de guidage aux os correspondants en position d'extension, une mise en flexion des os permet de désolidariser l'un ou les deux blocs de guidage de l'organe de stabilisation intra-articulaire. Cette opération permet de séparer les deux blocs de guidage afin de faciliter les opérations de coupes osseuses ultérieures.

Le dispositif chirurgical peut comprendre un organe apte à verrouiller la fixation des blocs de guidage sur l'organe de stabilisation intra-articulaire.

Avantageusement, pour faciliter la manipulation et la préhension de l'instrument, notamment pour aider à sa mise en place et son retrait, l'organe de verrouillage dudit instrument comprend une poignée.

Selon une forme de réalisation particulière, l'organe de stabilisation intra-articulaire présente une épaisseur variable afin de s'adapter au mieux à la morphologie du patient et de pouvoir ré-axer d'une manière optimale les deux os de l'articulation avant les opérations de coupes osseuses.

Selon une forme de réalisation particulière, l'organe de stabilisation intra-articulaire présente deux faces destinées à être en appui avec les os du patient, et dont au moins l'une des faces présente une forme complémentaire à l'os avec lequel elle est destinée à être en appui. De cette manière, le dispositif selon l'invention est sur mesure et s'adapte parfaitement à la morphologie du patient.

Selon une autre forme de réalisation de l'organe de stabilisation intra-articulaire, celui-ci est constitué par un empilement de cales concaves ou convexes. Cette caractéristique permet de pouvoir ajuster l'épaisseur de l'organe de stabilisation en rajoutant ou en retirant une cale. Cela permet notamment de pouvoir s'adapter à la morphologie du patient et de ré-axer d'une manière optimale les deux os de l'articulation.

Avantageusement, les blocs de guidage forment une butée pour le positionnement de l'organe de stabilisation intra-articulaire. Plus précisément, les surfaces des blocs de guidage destinées à être en appui avec l'os, forment des butées. Ces surfaces d'appui sont, de préférence, adaptées à la forme des os avec lesquels elles sont destinées à être en appui.

L'invention vise à fournir un procédé de conception assistée par ordinateur d'un dispositif chirurgical d'aide à la pose d'un implant orthopédique entre deux os d'une articulation d'un patient.

Selon l'invention, le procédé de conception comprend des étapes consistant à :
- reconstruire, à partir d'imageries médicales, un modèle virtuel en trois dimensions de la portion d'articulation concernée ;
- corriger virtuellement l'alignement de l'articulation de sorte à définir un espace entre les deux os de ladite articulation correspondant à une partie usée à remplacer par l'implant ;
- positionner virtuellement dans l'espace du modèle virtuel de la portion d'articulation corrigée, un volume d'encombrement prothétique défini au moins entre deux plans ;
- concevoir virtuellement un organe de stabilisation intra-articulaire apte à remplir l'espace défini entre les deux os de l'articulation corrigée pour stabiliser et ré-axer l'articulation ;
- concevoir virtuellement deux blocs de guidage fixés, dont l'un au moins d'une manière amovible, à l'organe de stabilisation intra-articulaire et de part et d'autre d'un plan défini par ledit organe de stabilisation intra-articulaire, lesdits blocs de guidage comprenant chacun une fente coplanaire à un des deux plans définissant le volume d'encombrement prothétique.

Ainsi, le dispositif chirurgical d'aide à la pose d'un implant est conçu sur mesure et permet, d'une part, de ré-axer les deux os d'une articulation, sans coupes osseuses préalable, et, d'autre part, de positionner correctement les plans de coupes dans une position relative les uns par rapport aux autres. Après la mise en place du dispositif chirurgical entre les deux os de l'articulation, aucun ajustement de la balance ligamentaire n'est nécessaire, les coupes osseuses sont positionnées et peuvent être réalisées.

Avantageusement, le procédé comprend une étape consistant à fabriquer le dispositif chirurgical en fabriquant l'organe de stabilisation intra-articulaire et les deux blocs de guidage préalablement conçus, par une technique de fabrication additive.

Un procédé d'utilisation dudit dispositif chirurgical précité d'aide à la pose d'un implant orthopédique entre deux os d'une articulation d'un patient est divulgué, le procédé ne faisant pas partie de l'invention.

Ce procédé comprend des étapes consistant à :
- positionner l'organe de stabilisation intra-articulaire entre les deux os en extension de l'articulation de manière à stabiliser et aligner les os de ladite articulation ;
- fixer chaque bloc de guidage à un os de l'articulation ;
- mettre en flexion les deux os de l'articulation pour désolidariser au moins l'un des deux blocs de guidage de l'organe de stabilisation ;
- réaliser des opérations de coupes de parties des os au moyen d'un instrument guidé par les fentes des blocs de guidage.

De cette manière, l'utilisation du dispositif est simplifiée et permet de réaliser des coupes osseuses dans un environnement peu encombrant. En effet, les deux blocs de coupes sont positionnés simultanément et avec des plans de coupes positionnés relativement les uns par rapport aux autres, et sont ensuite rendus indépendants par une mise en flexion des deux os de l'articulation. Aucun ajustement de la balance ligamentaire ne nécessite d'être effectué, et les coupes osseuses sont précises.

D'autres caractéristiques et avantages de l'invention ressortiront clairement de la description qui en est réalisée ci-après, à titre indicatif et nullement limitatif, en référence aux figures annexées dans lesquelles :
- la figure 1 est une représentation schématique en perspective d'un dispositif chirurgical;
- la figure 2 est une représentation schématique illustrant le dispositif chirurgical vu de face ;
- la figure 3 est une représentation schématique illustrant le dispositif chirurgical vue de côté ;
- la figure 4 est une représentation schématique illustrant le dispositif chirurgical vu en perspective éclatée ;
- la figure 5 est une représentation schématique illustrant un modèle virtuel reconstruit en trois dimension d'une articulation usée et déformée avant la mise en place d'un implant ;
- la figure 6 est une représentation schématique illustrant le modèle virtuel de l'articulation de la figure 5, dont l'alignement a été corrigé virtuellement ;
- la figure 7 est un agrandissement du modèle virtuel de l'articulation de la figure 6, de manière à illustrer l'espace ainsi défini entre les deux os de l'articulation virtuellement corrigée ;
- la figure 8 est une représentation schématique illustrant le dispositif chirurgical d'aide à la pose d'un implant orthopédique disposé virtuellement entre les deux os de l'articulation ;
- la figure 9 est une représentation schématique similaire à celle de la figure 8, illustrant les plans de coupes virtuellement positionnés relativement l'un par rapport à l'autre ;
- la figure 10 est une représentation schématique illustrant virtuellement l'articulation après la réalisation des coupes osseuses selon les plans de coupes ;
- la figure 11 est une représentation schématique illustrant un implant orthopédique mis en place après avoir réalisé les coupes osseuses.

On a illustré aux figures des dessins un exemple du dispositif de guidage adapté, par exemple, pour réaliser des coupes fémorales et tibiales sans pour cela exclure d'autres applications en fonction du type d'implant orthopédique à implanter, par exemple, un implant fémoral.

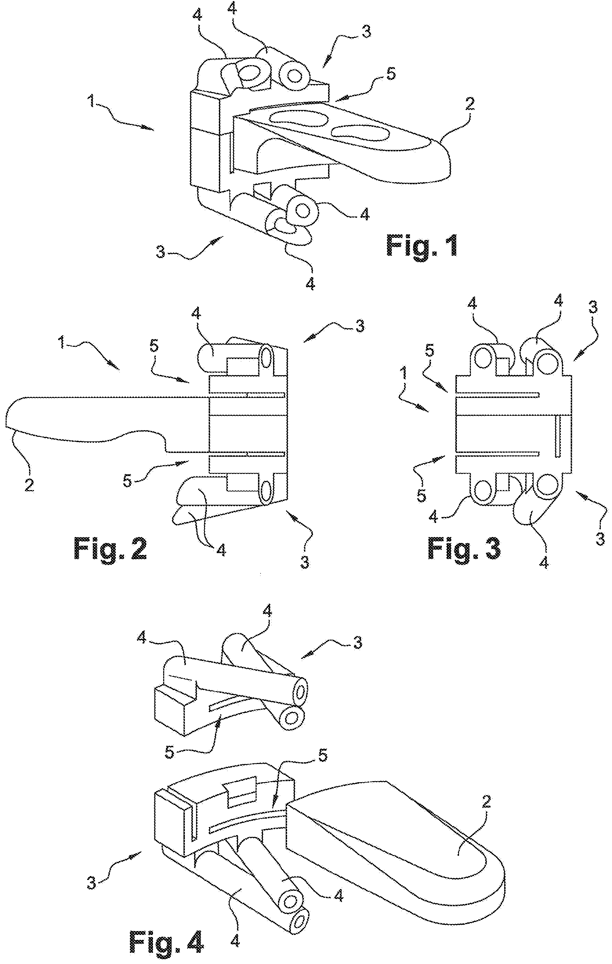

Quelle que soit la forme de réalisation, et en référence aux figures 1 à 4, le dispositif (1) comprend, d'une part, un organe de stabilisation intra-articulaire (2), et, d'autre part, deux blocs de guidage (3) d'un instrument destiné à réaliser des opérations de coupes de parties des os.

L'organe de stabilisation intra-articulaire (2) peut présenter diverses formes. En effet, l'essentiel réside dans le fait que l'organe de stabilisation intra-articulaire (2) est destiné à être positionné entre les deux os de l'articulation (6), notamment pour stabiliser et ré-axer le membre comprenant l'articulation (6), en comblant un espace (7) défini par l'usure d'une portion de l'articulation (6).

En référence à la forme de réalisation illustrée, l'organe de stabilisation intra-articulaire (2), généralement appelé « spacer », comprend deux faces, chacune destinées à être en appui avec un os de l'articulation (6), et défini de cette manière un plan principal.

L'une des faces du spacer (2), et de préférence les deux, peuvent présenter des formes complémentaires à l'os avec lesquelles elles sont destinées à être en appui. De cette manière, ledit spacer (2) est sur mesure et bien adapté à la morphologie du patient

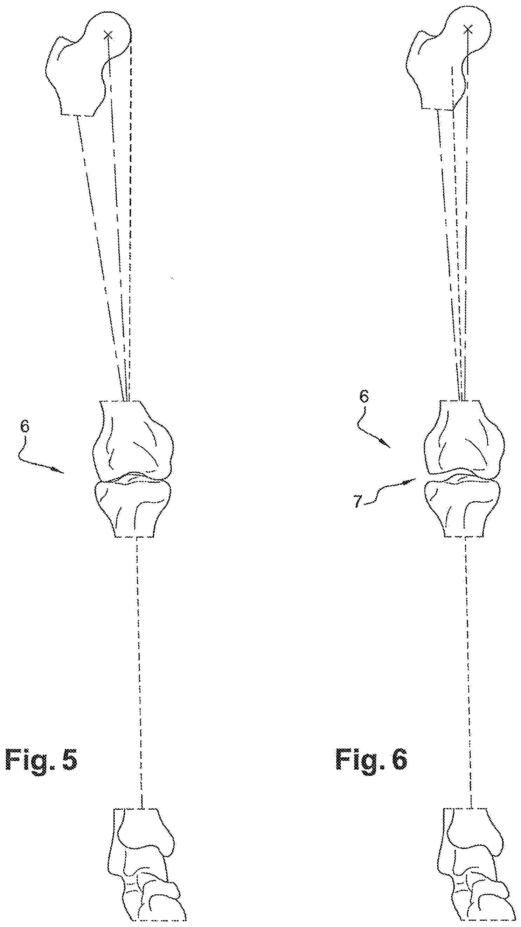

Pour concevoir un tel spacer (2) et en référence à la figure 5, il est nécessaire de reconstruire en premier lieu, et à partir d'imageries médicales, un modèle virtuel en trois dimensions de la portion d'articulation (6) concernée.

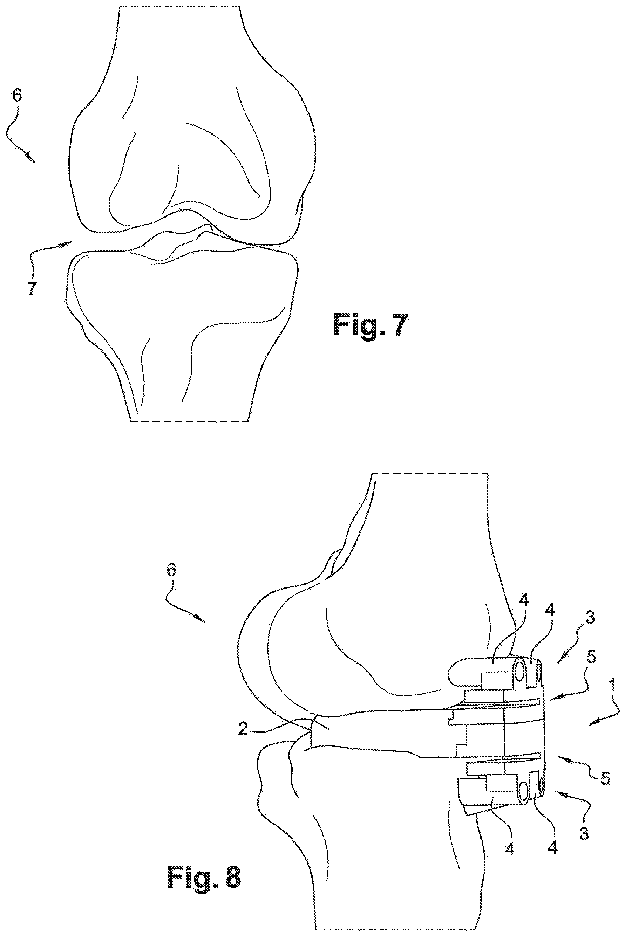

Cette portion d'articulation (6) nécessitant l'implantation d'une prothèse doit donc comprendre une partie usée et doit donc ne pas être correctement alignée. Une seconde étape consiste alors et en référence aux figures 6 et 7, à corriger virtuellement l'alignement de l'articulation (6) de sorte à définir un espace (7) entre les deux os de ladite articulation (6) correspondant à la partie usée à remplacer par un implant orthopédique (8).

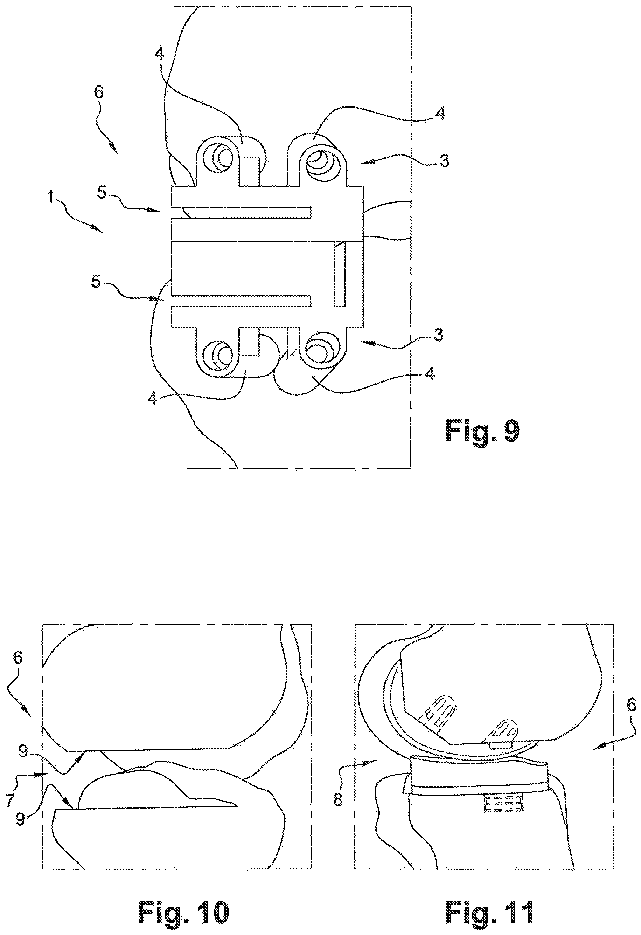

En référence aux figures 8 et 9, le spacer (2) est donc conçu d'une manière assistée par ordinateur par un ajout de matière virtuelle pour effectuer un remplissage de l'espace (7) défini entre les eux os de l'articulation (6). De cette manière, on obtient un spacer (2) de forme anatomique et sur mesure. Le positionnement ultérieur pendant l'intervention chirurgicale d'un tel spacer (2) entre les deux os de l'articulation (6) du patient permet ainsi de stabiliser et de ré-axer d'une manière optimale l'articulation (6) concernée.

Les blocs de guidage (3) sont fixés au spacer (2), avantageusement d'une manière amovible, et de part et d'autre du plan principal défini par ledit spacer (2). Les blocs de guidage (3) comprennent des moyens de fixation (4) à l'os, par exemple sous la forme de tubes orientés et aptes à recevoir chacun un moyen d'ancrage, telle qu'une broche lisse ou filetée. Ces tubes (4) s'étendent à partir des blocs de guidage (3) et comprennent une surface d'appui avec l'os. Cette surface d'appui permet de réaliser une butée lors du positionnement du spacer (2) entre les deux os de l'articulation (6). D'une manière avantageuse, la surface d'appui des tubes (4) présente une forme complémentaire à l'os avec lequel elle est destinée à être en appui pour assurer la stabilité rotatoire.

Dans un mode de réalisation avantageux et non représenté, les blocs de guidage (3) sont verrouillés dans leur position fixée sur le spacer (2) par l'intermédiaire d'un organe de verrouillage. Cet organe de verrouillage peut se présenter sous la forme d'une pince venant enserrer lesdits blocs de guidage (3) pour les maintenir en position fixée. La pince peut par exemple être prolongée par une poignée facilitant la manutention du dispositif chirurgical (1), et permettant ainsi de faciliter le positionnement du spacer (2) entre les deux os de l'articulation (6). La pince peut comprendre un système à encliquetage, par exemple, pour coopérer facilement avec lesdits blocs de guidage (3).

Les blocs de guidage (3) présentent ensuite chacun au moins une fente (5) pour l'engagement et le guidage d'un instrument destiné à réaliser des opérations de coupes des différentes parties osseuses.

La ou les fentes (5) de l'un des blocs de guidage (3) comprennent des orientations et des positions déterminées par rapport à la ou aux fentes (5) de l'autre bloc de guidage. Ainsi et en référence à la figure 9, les fentes (5) déterminent des plans de coupes, lesquels sont positionnés d'une manière relative les uns par rapport aux autres.

De la même manière que précédemment, les blocs de guidage (3) sont conçus d'une manière assistée par ordinateur. Le volume d'encombrement prothétique positionné dans l'espace (7) du modèle virtuel de l'articulation (6) vient en intersection avec les os dudit modèle virtuel. Les plans d'intersection définissent des plans de coupes osseuses à réaliser pour mettre en place l'implant orthopédique (8). Les blocs de guidage (3) sont conçus de manière à ce que leurs fentes (5) soient dans le prolongement de ces plans d'intersections. En d'autres termes, les fentes (5) des blocs de guidage (3) sont coplanaires avec lesdits plans de coupes définis par le volume d'encombrement prothétique.

Ainsi la conception assistée par ordinateur permet de concevoir sur mesure ces blocs de guidage (3). En effet, les surfaces d'appui des blocs de guidage (3) ainsi que l'extrémité des tubes (4) sont conçues pour être de formes complémentaires avec les os sur lesquels elles sont en appui. De plus, les fentes (5) des blocs de guidage (3) sont positionnées pour permettre de réaliser des coupes osseuses selon les plans de coupes définis par le volume d'encombrement prothétique.

Lorsque les blocs de guidage (3) et le spacer (2) sont conçus d'une manière virtuelle, il convient de programmer leur fabrication par des techniques bien connues de l'état de la technique, telle que par impression 3D directe ou indirecte.

Les blocs de guidage (3) peuvent être fixés de toute manière appropriée au spacer (2), et peuvent même former qu'une seule pièce avec ledit spacer (2), ce dispositif ne faisant pas partie de l'invention. Au moins l'un des blocs de guidage (3), et de préférence les deux, est fixé d'une manière amovible audit spacer (2) pour faciliter les opérations de coupes comme il sera décrit ci-après.

En effet, lors de l'utilisation du dispositif chirurgical (1) d'aide à la mise en place d'un implant orthopédique (8) entre deux os d'une articulation (6) d'un patient, il convient tout d'abord de positionner le spacer (2) entre les deux os en extension de l'articulation (6). Comme vu ci-avant, le positionnement du spacer (2) permet de stabiliser et de ré-axer l'articulation (6). Cette mise en place peut être effectuée en maintenant ledit spacer (2) par la poignée de l'organe de verrouillage du type pince. Le spacer (2) est positionné jusqu'à ce que les blocs de guidage (3), et notamment leurs tubes de fixation (4), viennent en butée contre les os de l'articulation (6).

Ensuite, il convient de fixer chaque bloc de guidage (3) à l'os correspondant de l'articulation (6). Cette étape est réalisée en insérant des broches lisses ou filetées dans chaque tube de fixation (4), et en les solidarisant à l'os de l'articulation (6), par exemple par vissage.

Ensuite, il convient de retirer la pince pour déverrouiller la fixation des blocs de guidage (3) sur le spacer (2). Ainsi, une mise en flexion des deux os permet de désolidariser les deux blocs de guidage (3) du spacer (2). Comme évoqué plus haut, il suffit que l'un au moins des blocs de guidage (3) soit fixé d'une manière amovible au spacer (2) pour que cette opération soit réalisable. De préférence, les deux blocs de guidage (3) sont fixés d'une manière amovible au spacer (2) pour faciliter les opérations de coupes ultérieures.

Il reste ensuite à réaliser des opérations de coupes en tant que telles de parties des os par l'intermédiaire d'un instrument à lame, guidé par les fentes (5) des blocs de guidage (3).

En référence à la figure 10, les coupes osseuses (9) permettent de préparer les os pour recevoir, en référence à la figure 11, l'implant orthopédique (8) proprement dit.

Le dispositif chirurgical (1) selon l'invention permet donc de positionner simultanément les plans de coupes osseuses de telle façon que la position relative des coupes sera parfaite et correctement balancée ce qui assure une restitution optimale de l'interligne articulaire.

## Revendications

1. Procédé de conception assistée par ordinateur d'un dispositif chirurgical (1) d'aide à la pose d'un implant orthopédique (8) entre deux os d'une articulation (6) d'un patient, comprenant des étapes consistant à :
- reconstruire, à partir d'imageries médicales, un modèle virtuel en trois dimensions de la portion d'articulation (6) concernée ;
- corriger virtuellement l'alignement de l'articulation (6) de sorte à définir un espace (7) entre les deux os de ladite articulation (6) correspondant à une partie usée à remplacer par l'implant ;
- positionner virtuellement dans l'espace (7) du modèle virtuel de la portion d'articulation (6) corrigée, un volume d'encombrement prothétique défini au moins entre deux plans ;
- concevoir virtuellement un organe de stabilisation intra-articulaire (2) apte à remplir l'espace (7) défini entre les deux os de l'articulation (6) corrigée pour stabiliser et ré-axer l'articulation (6) ;
- concevoir virtuellement deux blocs de guidage (3) fixés à l'organe de stabilisation intra-articulaire (2) et de part et d'autre d'un plan défini par ledit organe de stabilisation intra-articulaire (2), lesdits blocs de guidage (3) comprenant chacun une fente (5) coplanaire à un des deux plans définissant le volume d'encombrement prothétique;
**caractérisé en ce qu'**au moins l'un des deux blocs de guidage (3) est fixé à l'organe de stabilisation intra-articulaire (2) d'une manière amovible.

2. Procédé selon la revendication 1, ***caractérisé* en ce que** les deux blocs de guidage sont conçus de sorte à être fixés d'une manière amovible à l'organe de stabilisation intra-articulaire (2).

3. Procédé selon la revendication 1, ***caractérisé* en ce qu'**il comprend une étape consistant à concevoir virtuellement un organe apte à verrouiller la fixation des blocs de guidage (3) sur l'organe de stabilisation intra-articulaire (2).

4. Procédé selon la revendication 1, ***caractérisé* en ce que** l'organe de stabilisation intra-articulaire (2) est conçu de sorte à présenter une épaisseur variable.

5. Procédé selon la revendication 1, ***caractérisé* en ce que** l'organe de stabilisation intra-articulaire (2) est conçu de sorte à présenter deux faces destinées à être en appui avec les os du patient, et dont au moins l'une des faces présente une forme complémentaire à l'os avec lequel elle est destinée à être en appui.

6. Procédé selon la revendication 4, ***caractérisé* en ce que** l'organe de stabilisation intra-articulaire (2) est conçu de sorte à être constitué par un empilement de cales concaves ou convexes.

7. Procédé selon la revendication 1, ***caractérisé* en ce que** les blocs de guidage (3) sont conçus de sorte à former une butée pour le positionnement de l'organe de stabilisation intra-articulaire (2).

8. Procédé selon la revendication 1, ***caractérisé* en ce qu'**il comprend une étape consistant à fabriquer le dispositif chirurgical (1) en fabriquant l'organe de stabilisation intra-articulaire (2) et les deux blocs de guidage (3) préalablement conçus, par une technique de fabrication additive.

## Patentansprüche

1. Computergestütztes Entwurfsverfahren eines chirurgischen Instruments (1) zur Unterstützung bei der Platzierung eines orthopädischen Implantats (8) zwischen zwei Knochen eines Gelenks (6) eines Patienten, umfassend die folgenden Schritte:
- Rekonstruktion, aus medizinischen Bildern, eines dreidimensionalen virtuellen Modells des betreffenden Gelenkabschnitts (6);
- virtuelle Korrektur der Ausrichtung des Gelenks (6) derart, dass ein Raum (7) zwischen den beiden Knochen des Gelenks (6) definiert wird, der einem abgenutzten, durch das Implantat zu ersetzenden Abschnitt entspricht;
- virtuelle Positionierung in dem Raum (7) des virtuellen Modells des korrigierten Gelenkabschnitts (6), wobei ein prothetisches Raumvolumen mindestens zwischen zwei Ebenen definiert ist;
- virtuelle Gestaltung eines intraartikulären Stabilisierungselements (2), das in der Lage ist, den zwischen den beiden Knochen des Gelenks (6) definierten Raum (7) zu füllen, der korrigiert wurde, um das Gelenk (6) zu stabilisieren und umzulenken;
- virtueller Entwurf von zwei Führungsblöcken (3), die am intraartikulären Stabilisierungselement (2) befestigt sind, und auf beiden Seiten einer Ebene, die durch das intraartikuläre Stabilisierungselement (2) definiert ist, wobei die Führungsblöcke (3) jeweils einen Schlitz (5) umfassen, der koplanar zu einer der beiden Ebenen ist, die das prothetische Raumvolumen definieren; ***dadurch gekennzeichnet,* dass** mindestens einer der beiden Führungsblöcke (3) lösbar am intraartikulären Stabilisierungselement (2) befestigt ist.

2. Verfahren nach Anspruch 1, ***dadurch gekennzeichnet,* dass** die beiden Führungsblöcke so ausgelegt sind, dass sie lösbar am intraartikulären Stabilisierungselement (2) befestigt werden.

3. Verfahren nach Anspruch 1, ***dadurch gekennzeichnet,* dass** es einen Schritt des virtuellen Entwerfens eines Elements umfasst, das in der Lage ist, die Befestigung der Führungsblöcke (3) an dem intraartikulären Stabilisierungselement (2) zu verriegeln.

4. Verfahren nach Anspruch 1, ***dadurch gekennzeichnet,* dass** das intraartikuläre Stabilisierungselement (2) so ausgelegt ist, dass es eine variable Dicke aufweist.

5. Verfahren nach Anspruch 1, ***dadurch gekennzeichnet,* dass** das intraartikuläre Stabilisierungselement (2) so ausgelegt ist, dass es zwei Flächen aufweist, die dazu bestimmt sind, an dem Knochen des Patienten anzuliegen, und von denen mindestens eine Fläche eine Form aufweist, die zu dem Knochen komplementär ist, an dem sie zum Anliegen bestimmt ist.

6. Verfahren nach Anspruch 4, ***dadurch gekennzeichnet,* dass** das intraartikuläre Stabilisierungselement (2) dazu bestimmt ist, durch einen Stapel von konkaven oder konvexen Keilen gebildet zu werden.

7. Verfahren nach Anspruch 1, ***dadurch gekennzeichnet,* dass** die Führungsblöcke (3) so ausgebildet sind, dass sie einen Anschlag für die Positionierung des intraartikulären Stabilisierungselements (2) bilden.

8. Verfahren nach Anspruch 1, ***dadurch gekennzeichnet,* dass** es einen Schritt zur Herstellung des chirurgischen Instruments (1) durch Herstellung des intraartikulären Stabilisierungselements (2) und der beiden zuvor entwickelten Führungsblöcke (3) durch eine additive Fertigungstechnik umfasst.

## Claims

1. A computer-assisted design method for a surgical device (1) to assist with the placement of an orthopedic implant (8) between two bones of a joint (6) of a patient, including steps consisting of:
- reconstructing, from medical images, a three-dimensional virtual model of the joint portion (6) in question;
- virtually correcting the alignment of the joint (6) so as to define a space (7) between the two bones of said joint (6) corresponding to a worn part to be replaced with the implant;
- virtually positioning, in the space (7) of the virtual model of the corrected joint portion (6), a prosthetic space consumption defined at least between two planes;
- virtually designing an intra-articular stabilization member (2) able to fill the space (7) defined between the two bones of the corrected joint (6) in order to stabilize and realign the joint (6);
- virtually designing two guide blocks (3) fastened to the intra-articular stabilization member (2) on either side of a plane defined by said intra-articular stabilization member (2), said guide blocks (3) each including a slit (5) coplanar with respect to one of the two planes defining the prosthetic space consumption;
***characterized* in that** at least one of the two guide blocks (3) is fastened to the intra-articular stabilization member (2) removably.

2. The method according to claim 1, ***characterized* in that** the two guide blocks are designed so as to be fastened removably to the intra-articular stabilization member (2).

3. The method according to claim 1, ***characterized* in that** it includes a step consisting of virtually designing a member capable of locking the fastening of the guide blocks (3) on the intra-articular stabilization member (2).

4. The method according to claim 1, ***characterized* in that** the intra-articular stabilization member (2) is designed so as to have a variable thickness.

5. The method according to claim 1, ***characterized* in that** the intra-articular stabilization member (2) is designed so as to have two faces intended to support the bones of the patient, and at least one of the faces of which has a shape complementary to the bone on which it is intended to support.

6. The method according to claim 4, ***characterized* in that** the intra-articular stabilization member (2) is designed so as to be made up of a stack of concave or convex shims.

7. The method according to claim 1, ***characterized* in that** the guide blocks (3) are designed so as to form a stop for positioning the intra-articulation stabilization member (2).

8. The method according to claim 1, ***characterized* in that** it includes a step consisting of manufacturing the surgical device (1) by manufacturing the intra-articulation stabilization member (2) and the two guide blocks (3) designed beforehand, using an additive manufacturing technique.
